# EUROPEAN PATENT APPLICATION

(11) **EP 3 991 793 A1**
(43) Date of publication of application: **04.05.2022**
(21) Application number: 20833610.7
(22) Date of filing: 26.06.2020
(51) Int. Cl.: A61P 3/10, C12N 5/077, A61K 35/39

(54) **METHOD FOR SEPARATING PANCREATIC PROGENITOR CELLS**

(30) Priority: 27.06.2019 JP 2019119555
(71) Applicant: The University of Tokyo, Bunkyo-ku Tokyo 113-8654 (JP)
(72) Inventor: MIYAJIMA Atsushi, Tokyo 113-8654 (JP); WATANABE Ami, Tokyo 113-8654 (JP)
(74) Representative: Vossius & Partner Patentanwälte Rechtsanwälte mbB
(86) International application number: PCT/JP2020/025230
(87) International publication number: WO 2020/262613

(57) **Abstract**

It is an object of the present invention to provide a method for preparing or separating pancreatic progenitor cells (or a group of pancreatic progenitor cells) that do not contain undifferentiated cells and efficiently differentiate into pancreatic islet cells. More specifically, the present invention relates to: pancreatic progenitor cells that appear in the process of differentiation of stem cells into pancreatic islet cells, which are characterized in that the pancreatic progenitor cells are positive to CD82; an agent for treating blood glucose level impairment, wherein the agent comprises the pancreatic progenitor cells; and a method for preparing pancreatic progenitor cells, wherein the method comprises separating CD82-positive cells.

## Description

### Technical Field

The present invention relates to a method for separating pancreatic progenitor cells.

### Background Art

Replenishment of pancreatic endocrine cells (α cells secreting glucagon, β cells secreting insulin, δ cells secreting somatostatin, ε cells secreting ghrelin, and the like) by transplantation of pancreatic islets (or islets of Langerhans) is effective for the treatment of severe diabetes. However, due to lack of donor pancreas, this treatment method has not been widely used. Thus, in order to obtain a large number of pancreatic islets, a method for preparing pancreatic islet cells (α cells, β cells, δ cells, ε cells, PP cells, and the like) *in vitro* from pluripotent stem cells such as ES/iPS cells has attracted attention as an alternative method, and at present, studies regarding this method has been promoted (Non Patent Literatures 1 to 6, etc.).

Induction of differentiation of pluripotent stem cells into pancreatic islet cells is carried out by imitating various types of signal transductions generated in the pancreas development process. In recent years, the detailed analysis of gene expression by the analysis of the pancreas development process using mouse models and elucidation of the signal transduction mechanism have been progressed, and as a result, the efficiency of inducing *in vitro* differentiation of pluripotent stem cells into pancreatic islet cells has been drastically improved. Nevertheless, elucidation of signals important for the development of the pancreas and the maturation thereof has not been perfect yet, and many points still remain to be elucidated. In particular, there are many unclear points regarding the process of maturing the insulin secretion function of β cells.

Moreover, the recently reported results of single-cell gene analysis have demonstrated that there is a large difference in terms of gene expression between human fetus/adult pancreatic islets, and pancreatic islet cells whose differentiation has been induced by a conventional method (Non Patent Literature 7 and Non Patent Literature 8). This fact shows that important findings regarding the development process of pancreas have not yet been known.

In view of the above, it is doubtful whether completely mature cells could be obtained at the same level as *in vivo* according to the previously reported *in vitro* differentiation induction method. In order to obtain *in vitro* mature pancreatic islet cells, in particular, pancreatic β cells, it becomes important to analyze, in more detail, signals that are essential for the process of the development of the pancreas, in particular, the process of the development of human pancreatic islets.

However, at present, the process of the development of pancreatic islets has not fully elucidated yet. In addition, a method of efficiently inducing differentiation of stem cells into pancreatic islet cells *in vitro* has not been established, either.

Moreover, if stem cells are induced to differentiate into pancreatic islet cells according to a conventional method, it is inevitable that cells that have not differentiated into pancreatic islet cells are mixed into differentiated cells. Thus, there has been a risk that when the differentiated cells are transplanted into a living body, the thus mixed undifferentiated cells may become cancerous (Non Patent Literature 2, Non Patent Literature 3, etc.). Hence, it is an important object in the field of regenerative medicine to establish a method of efficiently inducing differentiation of stem cells into pancreatic progenitor cells, in which the mixing of undifferentiated cells is avoided.

### Citation List

### Non Patent Literature

Non Patent Literature 1: D'Amour et al., Nat Biotechnol. 24: 1392-1401, 2006.
Non Patent Literature 2: Kroon et al., Nat Biotechnol. 26: 443-452, 2008.
Non Patent Literature 3: Rezania et al., Diabetes. 61: 2016-2029, 2012.
Non Patent Literature 4: Rezania et al., Nat Biotechnol. 32: 1121-1133, 2014.
Non Patent Literature 5: Pagliuca et al., 159: 428-439, 2014.
Non Patent Literature 6: Russ et al., EMBO J. 34: 1759-1772, 2015.
Non Patent Literature 7: Ramond et al., Development 145, dev165480. 2018.
Non Patent Literature 8: Petersen et al., Stem Cell Reports 9: 1246-1261, 2017.

### Summary of Invention

### Technical Problem

In view of the aforementioned circumstances, it is an object of the present invention to provide a method for preparing or separating pancreatic progenitor cells (or a group of pancreatic progenitor cells) that do not contain undifferentiated cells and efficiently differentiate into pancreatic islet cells.

### Solution to Problem

To date, it had been reported that the cells strongly expressing Ngn3, an endocrine progenitor cell marker, differentiate into pancreatic endocrine cells in an *in vitro* differentiation induction system (H Liu et al., Cell Res. Oct;24(10): 1181-200. 2014).

Thus, the present inventors have attempted to search for a novel pancreatic progenitor cell-specific marker, by using the expression level of Ngn3 as an indicator. As a result, the inventors have discovered CD82. The present inventors have confirmed that the cells that have been sorted by using CD82 expression as an indicator efficiently differentiate into pancreatic islet cells, in particular, pancreatic β cells, in the process of differentiation of pluripotent stem cells into pancreatic islet cells. The sorted CD82 cell-derived pancreatic islet cells showed Glucose-stimulated insulin secretion *in vitro.* Furthermore, from these findings, it has been revealed that pancreatic endocrine cells that do not contain undifferentiated cells can be efficiently obtained, if CD82-positive cells appearing in the process of differentiation of stem cells into pancreatic islet cells are purified *in vitro* and then, a maturation culture of the cells into pancreatic β cells are carried out.

Specifically, the present invention includes the following (1) to (7).
(1) Pancreatic progenitor cells appearing in the process of differentiation of stem cells into pancreatic islet cells, which are characterized in that the pancreatic progenitor cells are positive to CD82.
(2) Pancreatic progenitor cells according to the above (1), which are characterized in that the stem cells are pluripotent stem cells.
(3) A cell group comprising the pancreatic progenitor cells according to the above (1) or (2).
(4) An agent for treating blood glucose level impairment, wherein the agent comprises the pancreatic progenitor cells according to the above (1) or (2).
(5) The treatment agent according to the above (4), which is characterized in that the blood glucose level impairment is hyperglycemia.
(6) A method for preparing pancreatic progenitor cells and/or mature pancreatic β cells, wherein the method comprises separating CD82-positive cells that appear in the process of differentiation of stem cells into pancreatic islet cells.
(7) The method according to the above (6), which is characterized in that the stem cells are pluripotent stem cells.

### Advantageous Effects of Invention

The pancreatic progenitor cells of the present invention are purified (i.e. are separated from other cell species) and are then subjected to a maturation culture into individual types of pancreatic islet cells (e.g. β cells, etc.), so that undifferentiated cells can be removed. Therefore, the pancreatic progenitor cells (or cell group) of the present invention are used as a material for transplantation of pancreatic islets, so that malignant conversion caused by the mixing of undifferentiated cells can be avoided and the improvement of the safety of transplantation therapy can be expected.

By purifying the pancreatic progenitor cells of the present invention, unnecessary cells are removed, and the efficiency of the maturation culture of desired cells is improved. Therefore, according to the present invention, it becomes possible to obtain pancreatic endocrine cells with high efficiency.

### Brief Description of Drawings

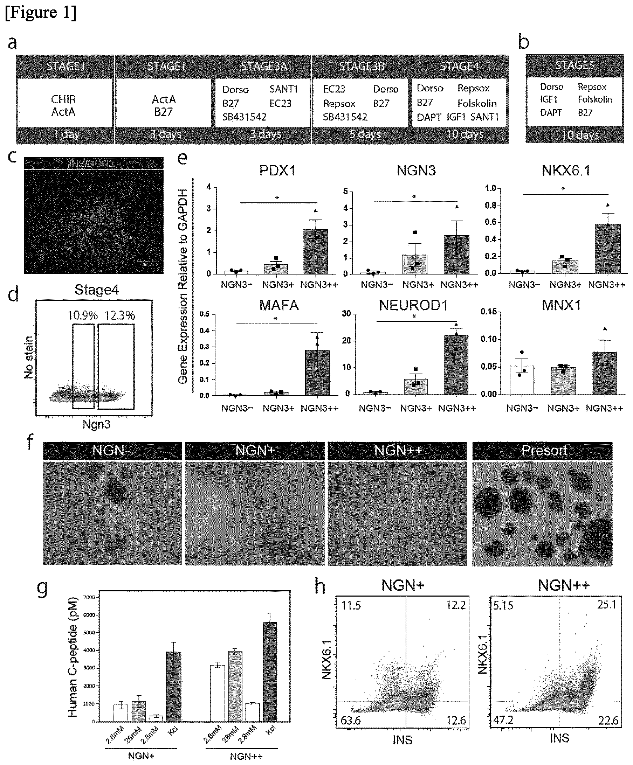

[Figure 1] Figure 1 shows the results obtained by studying induction of differentiation of Ngn3-high expressing cells into pancreatic endocrine cells. Figure 1(a) shows a scheme for differentiation of human iPS cells into pancreatic endocrine progenitor cells. Figure 1(b) shows maturation protocols of pancreatic endocrine cells. Figure 1(c) is a view showing the expression state of reporter genes in EP stage cells. Ngn3-mcherry and INS-venus were detected using a fluorescence microscope. The scale bar is 200 µm. Figure 1(d) shows the results obtained by analyzing EP stage cells derived from Ngn3 reporter iPS cells according to flow cytometry. Figure 1(e) shows the results obtained by analyzing the sorted Ngn3-negative cells (NGN-), Ngn3-Low expressing cells (NGN+) and Ngn3-high expressing cells (NGN++) according to a qRT-PCR method. The expression levels of individual genes are normalized by GAPDH. The data are shown as a mean value ±SEM (N = 3). Figure 1(f) shows bright field images of Ngn3-negative cells, Ngn3-low expressing cells, Ngn3-high expressing cells, and non-sorted cells (Presort), on Day 32 of the culture. The scale bar is 200 µm. Figure 1(g) shows the results obtained by measuring according to ELISA, human C-peptides secreted *in vitro* from individual cell clusters, namely, Ngn3-Lowexpressing cells (NGN+) and Ngn3-high expressing cells (NGN++). Figure 1(h) shows the results obtained by analyzing according to flow cytometry, the cell clusters derived from Ngn3-Low expressing cells (NGN+) and Ngn3-high expressing cells (NGN++), which were coimmunostained with an anti-Nkx6.1 antibody and an anti-INS antibody.

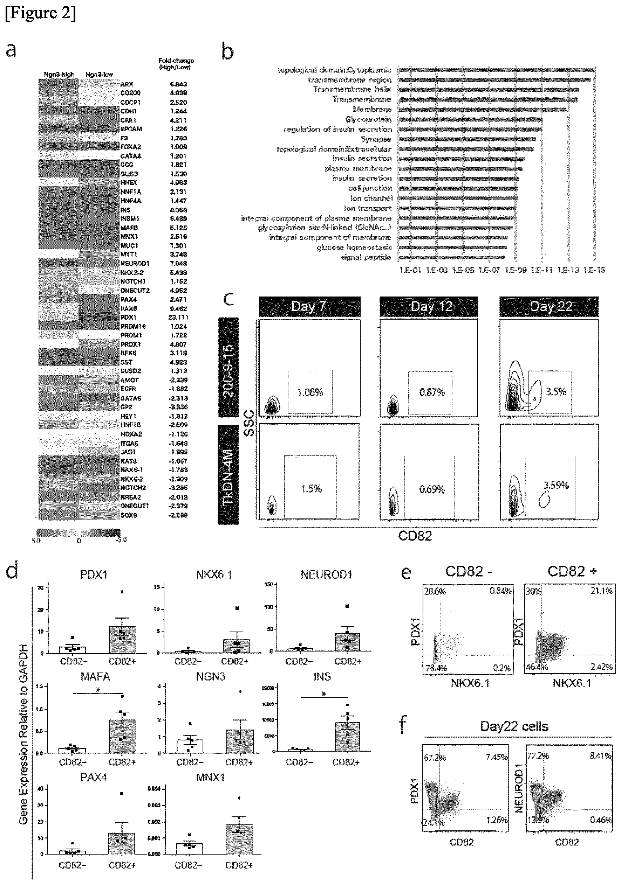

[Figure 2] Figure 2 shows the results obtained by studying the functions of CD82 as a pancreatic endocrine progenitor cell marker. Figure 2(a) shows a heat map of pancreatic progenitor genes expressed in Ngn3-high expressing cells (left, Ngn3-high) and Ngn3-low expressing cells (right, Ngn3-low). The scale indicates a normalized expression value. Figure 2 (b) shows the results of a GO analysis showing the condensation of genes in Ngn3-high expressing cells. Representative GO categories are shown and plotted against logarithm (P value). Figure 2(c) shows the results obtained by analyzing according to flow cytometry, CD82-expressing cells on Day 7, Day 12, and Day 22 after induction of differentiation of the cells. Figure 2(d) shows the results of a qRT-PCR analysis performed on CD82-negative cells (CD82-) and CD82-positive cells (CD82+) sorted from pancreatic progenitor cells. Expression levels are normalized by GAPDH. The data are shown as a mean value ±SEM (N = 3). Figure 2(e) shows the results obtained by analyzing according to flow cytometry, CD82-positive cells (CD82+) and CD82-negative cells (CD82-), which were coimmunostained with an anti-PDX1 antibody and an anti-NKX6.1 antibody. Figure 2(f) shows the results obtained by analyzing according to flow cytometry, EP cells on the 22nd day after the culture (Day 22), which was coimmunostained with an anti-CD82 antibody and an anti-PDX1 antibody, and with an anti-CD82 antibody and an anti-NEUROD1 antibody.

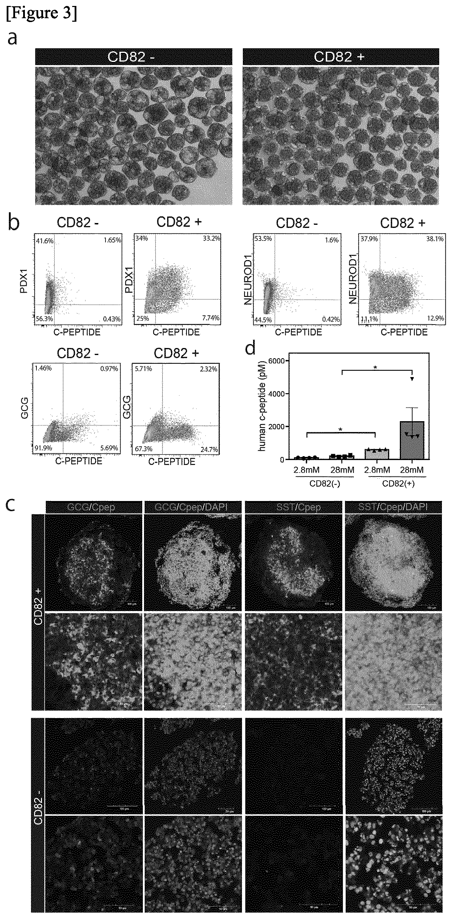

[Figure 3] Figure 3 shows the results obtained by studying induction of differentiation of CD82-positive cells into mature pancreatic endocrine cells. Figure 3(a) shows bright field images of cell clusters derived from CD82-positive cells (CD82+) and CD82-negative cells (CD82-). The scale bar is 100 µm. Figure 3(b) shows the results obtained by analyzing according to flow cytometry, a CD82-positive cell cluster (CD82+) and CD82-negative cell cluster (CD82-), which were coimmunostained with an anti-PDX1 antibody and an anti-C-peptide antibody, with an anti-NEUROD1 antibody and an anti-C-peptide antibody, and with an anti-GCG (glucagon) antibody and an anti-C-peptide antibody. Figure 3(c) shows the results obtained by coimmunostaining cell clusters derived from CD82-positive cells (CD82+) and CD82-negative cells (CD82-) with each of an anti-GCG antibody (red)/an anti-C-peptide antibody (green), an anti-GCG antibody (red)/an anti-C-peptide antibody (green)/DAPI (blue), an anti-SST (somatostatin) antibody (red)/an anti-C-peptide antibody (green), and an anti-SST (somatostatin) antibody (red)/an anti-C-peptide antibody (green)/DAPI (blue). Figure 3(d) shows the results obtained by measuring according to ELISA, human C-peptides secreted from a CD82-positive cell cluster (CD82+) and a CD82-negative cell cluster (CD82-). The data are shown as a mean value ±SEM (N = 4).

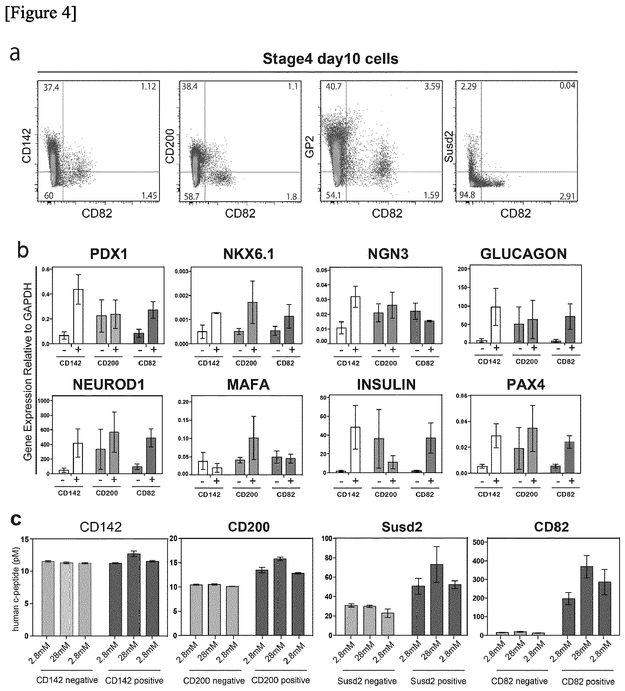

[Figure 4] Figure 4 shows the results obtained by studying a comparison of CD82 with known pancreatic endocrine cell markers. Figure 4(a) shows the results obtained by analyzing according to flow cytometry, EP cells on the 22nd day after the culture (Day 22), which were coimmunostained with each of an anti-CD142 antibody/an anti-CD82 antibody, an anti-CD200 antibody/an anti-CD82 antibody, an anti-GP2 antibody/an anti-CD82 antibody, and an anti-Susd2 antibody/an anti-CD82 antibody. Figure 4(b) shows the results obtained by measuring according to a qRT-PCR method, the expression levels of the previously reported EP marker genes (PDX1, NKX6.1, NGN3, GLUCAGON, NEUROD1, MAFA, INSULIN, and PAX4.1) in CD142-positive cells, CD200-positive cells, and CD82-positive cells. Figure 4(c) shows the results obtained by measuring according to ELISA, the amounts of human C-peptides secreted *in vitro* from each of CD142-positive cells/-negative cells, CD200-positive cells/-negative cells, Susd2-positive cells/-negative cells, and CD82-positive cells/-negative cells. The data are shown as a mean value ±SEM (N = 3).

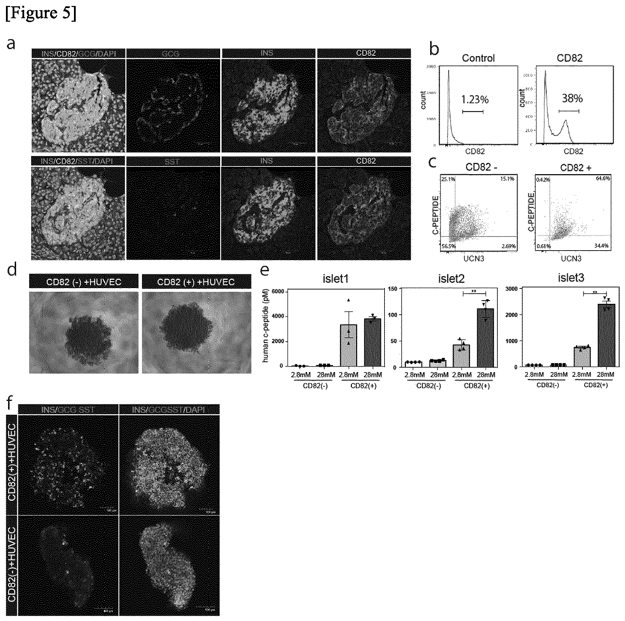

[Figure 5] Figure 5 shows the results obtained by studying the characteristics of CD82-positive cells in human mature pancreatic islets. Figure 5(a) shows the results obtained by immunostaining human pancreas-derived tissue sections with an anti-GCG antibody (red), an anti-SST antibody (red), an anti-INS antibody (green), an anti-CD82 antibody (gray), and DAPI. The scale bar is 50 µm. Figure 5(b) shows the results obtained by immunostaining the isolated human pancreatic islets with an anti-CD82 antibody, and then analyzing them according to flow cytometry. Figure 5(c) shows the results obtained by analyzing according to flow cytometry, CD82-positive cells and CD82-negative cells derived from human pancreatic islets, which were immunostained with an anti-C-peptide antibody and an anti-UCN3 antibody. Figure 5(d) shows bright field images of cell clusters prepared by co-culturing the isolated CD82-positive cells/- negative cells and HUVEC cells. Figure 5(e) shows the GSIS measurement results of the cell clusters derived from human pancreatic islets, which were prepared in Figure 5(d). The results of the pancreatic islets of three representative donors are shown. The data are shown as a mean value ±SEM (N = 3 to 4). Figure 5(f) shows the results obtained by staining the cell clusters derived from human pancreatic islets with an anti-INS antibody (green), an anti-GCG antibody (red), an anti-SST antibody (red), and DAPI (blue).

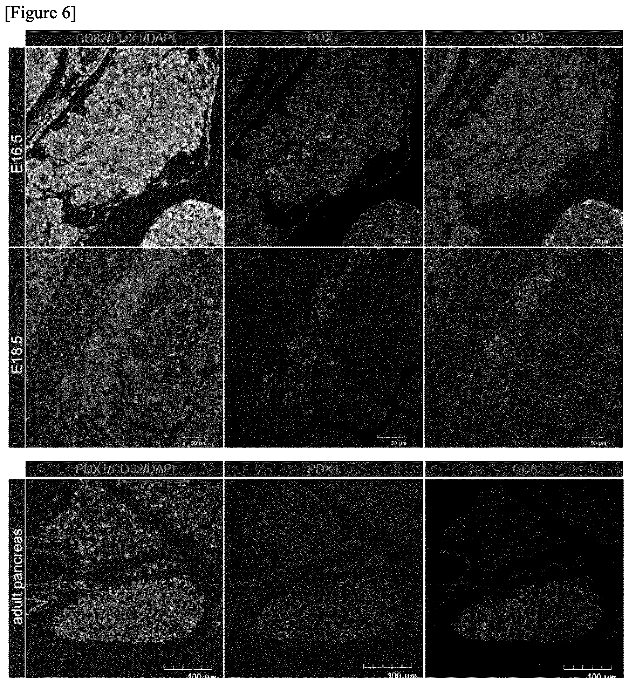

[Figure 6] Figure 6 shows the results obtained by studying the expression period of CD82 *in vivo.* The upper panels show the results obtained by immunostaining mouse fetus-derived pancreatic tissues (E16.5 and E18.5) with an anti-CD82 antibody (green), PDX1 (red), and DAPI (blue). The scale bar is 50 µm. The lower panels show the results obtained by immunostaining adult mouse-derived pancreatic tissues with an anti-CD82 antibody (green), PDX1 (red), and DAPI (blue). The scale bar is 100 µm.

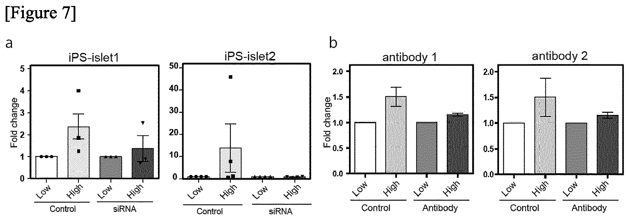

[Figure 7] Figure 7 shows the results obtained by studying the influence of CD82 on the insulin secretion of β cells. Figure 7(a) shows the results obtained by knocking down CD82 in Ngn3-high expressing cells (two representative batches (iPS-isletl and iPS-islet2), n = 4) induced from human iPS cells, by using siRNA, and then evaluating GSIS. The value of a low glucose level is set at 1, and GSIS is indicated by the magnification thereof. The data are shown as a mean value ±SEM (N = 4). Figure 7(b) shows the results obtained by inhibiting the functions of CD82 in Ngn3-high expressing cells by using the CD82 inhibitory antibody 49F and a commercially available antibody (LSbio), and then evaluating GSIS. The value of a low glucose level is set at 1, and GSIS is indicated by the magnification thereof. The data are shown as a mean value ±SEM (N = 4).

### Description of Embodiments

A first embodiment of the present invention relates to pancreatic progenitor cells appearing in the process of differentiation of stem cells into pancreatic islet cells, which are characterized in that the pancreatic progenitor cells are positive for CD82 (hereinafter also referred to as "the pancreatic progenitor cells of the present invention").

In the present invention, the "pancreatic progenitor cells" mean cells that specifically differentiate into pancreatic islet cells. The "pancreatic islet cells" are cells that constitute a pancreatic islet, namely, the pancreatic islet cells are a generic term for α cells secreting glucagon, β cells secreting insulin, δ cells secreting somatostatin, ε cells secreting ghrelin, and F cells secreting pancreatic peptides. In the present embodiment, particularly preferred pancreatic progenitor cells are pancreatic progenitor cells that specifically differentiate into pancreatic β cells. The animal species, from which the pancreatic progenitor cells of the present invention are derived, is not particularly limited. Examples of the animal species may include animal species belonging to: rodents such as a rat, a mouse, and a Guinea pig; Lagomorpha such as a rabbit; Ungulate such as a bovine, a swine, a goat, and sheep; Carnivora such as a dog and a cat; and Primates such as a human, a monkey, a chimpanzee, a gorilla, and an orangutan. The animal species is particularly preferably a human.

The "CD82-positive" means that a cell expresses CD82 (cluster of differentiation 82) on the surface thereof. Whether or not a cell expresses CD82 can be confirmed by examining the presence of CD82 on the surface thereof according to a method using a molecule that specifically recognizes CD82 (e.g. an antibody or an aptamer (a peptide or a nucleic acid)), for example, according to flow cytometry, immunohistochemistry, a Western blotting method, an ELISA method, an RIA method, or a modified method thereof.

The "stem cells" according to each embodiment of the present invention are cells having both an ability to differentiate into various cells and a self-replicating ability. Depending on the differentiating ability, examples of the stem cells may include pluripotent stem cells (cells having an ability to differentiate into all types of tissues and cells that constitute a living body) and multipotent stem cells (cells having an ability to differentiate into, not all types, but several types of tissues and cells).

Examples of the pluripotent stem cells may include induced pluripotent stem cells (iPS cells), embryonic stem cells (ES cells), and embryonic germ cells (EG cells). The particularly preferred pluripotent stem cells may include iPS cells and ES cells. On the other hand, examples of the multipotent stem cells may include pancreatic stem cells capable of differentiating into various types of cells constituting the pancreas (see, for example, WO2017/156076; H Liu et al., Cell Res. Oct; 24(10): 1181-200. 2014; J Ameri et al., Cell Rep. Apr 4; 19(1): 36-49. 2017; OG Kelly et al., Nat Biotechnol. Jul 31; 29(8): 750-6. 2011; etc.), and cells that appear in the process of allowing pluripotent stem cells to differentiate into pancreatic islet cells.

The "iPS cells" can be produced by introducing several types of transcriptional factors (hereinafter referred to as "pluripotent factors") that impart pluripotency to somatic cells (e.g. fibroblasts, skin cells, etc.) into the somatic cells. As such pluripotent factors, many factors have already been reported, and thus, the pluripotent factors used herein are not limited. Examples of the pluripotent factors used herein may include the Oct family (for example, Oct3/4), the SOX family (for example, SOX2, SOX1, SOX3, SOX15, SOX17, etc.), the Klf family (for example, Klf4, Klf2, etc.), the MYC family (for example, c-MYC, N-MYC, L-MYC, etc.), NANOG, and LIN28. Regarding the method of establishing iPS cells, many publications have been issued. Thus, such publications can be referred to (see, for example, Takahashi et al., Cell 126: 663-676, 2006, Okita et al., Nature 448: 313-317, 2007, Wernig et al., Nature 448: 318-324, 2007, Maherali et al., Cell Stem Cell 1: 55-70, 2007, Park et al., Nature 451: 141-146, 2007, Nakagawa et al., Nat Biotechnol 26: 101-106, 2008, Wernig et al., Cell Stem Cell 2: 10-12, 2008, Yu et al., Science 318: 1917-1920, 2007, Takahashi et al., Cell 131: 861-872, 2007, and Stadtfeld et al., Science 322: 945-949, 2008, etc.).

The ES cells used in the present invention are not particularly limited, and in general, a fertilized egg in the blastocyst stage is cultured together with feeder cells, and then, proliferating inner cell cluster-derived cells are disintegrated, which are further sub-cultured. This operation is repeated, and finally, the obtained cells can be established as an ES cell line. Regarding the method for preparing ES cells, please refer to US5,843,780, US6,200,806, or the like, for example.

With regard to a method of inducing differentiation of the stem cells into pancreatic islet cells, a person skilled in the art could appropriately select an induction method suitable for purpose from known methods and methods obtained by modifying such known methods. Such a method of inducing differentiation of the stem cells into pancreatic islet cells is not particularly limited, and examples of the method may include the methods described in FW Pagliuca et al., Oct 9; 159(2): 428-39, 2014, HA Russ et al., EMBO J. Jul 2; 34(13): 1759-72. 2015, A Rezania et al., Nat Biotechnol. Nov; 32(11): 1121-33. 2014, and GG Nair et al., Nat Cell Biol. Feb; 21(2): 263-274, 2019., etc., and also, modified methods thereof.

The stem cells can be cultured in a suitable culture solution for animal cell culture (e.g. DMEM, EMEM, IMDM, PRMI 1640, F-12, etc.), to which necessary supplements (e.g. CHIR99021, B27, Dorsomorphin, EC23, SB431542, RepSox, IGF1, Folskolin, DAPT, etc.) are added at a suitable timing in the differentiation process. The temperature for culturing the stem cells could be appropriately selected by a person skilled in the art, and the culture temperature is, for example, 30°C to 40°C, and preferably, approximately 37°C.

The pancreatic progenitor cells of the present invention can be used in the form of a cell group (cell population) also as a treatment agent described later (a second embodiment of the present invention). Herein, the percentage of CD82-positive cells contained in the pancreatic progenitor cell group of the present invention is different depending on intended use. The percentage of CD82-positive cells contained in the present pancreatic progenitor cell group is, for example, 30% or more, 40% or more, 50% or more, 60% or more, 70% or more, 80% or more, 85% or more, preferably 90% or more, and more preferably 95% or more.

A second embodiment of the present invention relates to an agent for treating blood glucose level impairment, wherein the agent comprises the pancreatic progenitor cells of the present invention (hereinafter also referred to as "the treatment agent of the present invention").

The treatment agent of the present invention comprises, as active ingredients, the pancreatic progenitor cells of the present invention that specifically differentiate into pancreatic islet cells, and preferably comprises a cell group of the pancreatic progenitor cells at a high purity (at a purity of, for example, 80% or more, and preferably 90% or more). In addition, the treatment agent of the present invention may be administered to a patient in a state in which the treatment agent of the present invention comprises the pancreatic progenitor cells of the present invention, so that the pancreatic progenitor cells may be allowed to differentiate into pancreatic endocrine cells *in vivo.* Otherwise, the pancreatic progenitor cells may be induced to differentiate into pancreatic endocrine cells (pancreatic β cells, etc.) that are especially necessary for the treatment of the disease, and thereafter, the treatment agent of the present invention comprising the pancreatic endocrine cells may be administered to a patient. For example, when the recovery of both insulin secretion and glucagon secretion is intended, the treatment agent of the present invention may be administered to a patient in a state in which the treatment agent comprises pancreatic progenitor cells.

In the present description, the "blood glucose level impairment" is a concept including a state that is determined to be hyperglycemia or hypoglycemia in comparison to the average blood glucose level of healthy subjects, which is caused by the abnormality of pancreatic endocrine cells (due to dysfunction, a decrease in cell number, etc.), and a disease that becomes a cause of such a state or a result thereof, such as, for example, diabetes. The treatment agent of the present invention can be used for the transplantation of pancreatic islet cells. The transplantation of pancreatic islet cells can be carried out by injecting the treatment agent of the present invention into the portal vein, for example, in a state in which the treatment agent of the present invention comprises pancreatic progenitor cells, or in a state in which the treatment agent of the present invention comprises pancreatic endocrine cells (or pancreatic islet cells) that have been induced to differentiate from the pancreatic progenitor cells. In order to recover insulin secretion, glucagon secretion, and somatostatin secretion, it is effective to allow the pancreatic progenitor cells comprised in the treatment agent of the present invention to differentiate into pancreatic β cells, pancreatic α cells, and pancreatic δ cells, respectively, and then to transplant the obtained cells into the portal vein.

A third embodiment of the present invention relates to a method for treating blood glucose level impairment, wherein the method comprises administering the treatment agent of the present invention to a patient (hereinafter also referred to as "the treatment method of the present invention").

The term "treatment" is used herein to mean that, in a patient who has already been affected with blood glucose level impairment, progression and deterioration of the pathologic condition are inhibited or alleviated, and thereby, the treatment is carried out for the purpose of inhibiting or alleviating progression and deterioration of the abnormality of blood glucose levels. The term "treatment" used herein also includes a prophylactic treatment.

The target of the treatment method of the present invention is not particularly limited, as long as it is a mammal that is determined to be affected with blood glucose level impairment. A particularly preferred treatment target is a human.

A fourth embodiment of the present invention relates to a method for preparing pancreatic progenitor cells (or a cell group of pancreatic progenitor cells) and/or mature pancreatic β cells, wherein the method comprises separating CD82-positive cells that appear in the process of differentiation of stem cells into pancreatic islet cells.

Herein, the phrase "to separate CD82-positive cells" means that cells that do not express CD82 are removed, or CD82-positive cells are isolated, from a cell population comprising various cells. The thus separated CD82-positive cells have the properties of pancreatic progenitor cells, by which the CD82-positive cells differentiate into pancreatic islet cells (β cells, α cells, etc.) as a result of being subjected to a maturation culture. Moreover, the CD82-positive cells separated from mature pancreatic islet cells are excellent in terms of insulin secretion, compared with CD82-negative cells, and CD82 can also be used as a marker for separating mature pancreatic β cells after completion of the maturation culture.

Isolation of the CD82-positive cells can be easily carried out by directly or indirectly labeling a molecule that specifically recognizes CD82 (for example, an antibody, or an aptamer (a peptide or a nucleic acid)) (wherein the labeling substance is not particularly limited, and a fluorescent molecule, a radioactive molecule, etc. can be used), and then by applying a method that is well known to a person skilled in the art, such as a FACS (Fluorescence activated cell sorting) or MACS (Magnetic cell sorting) method.

The period, in which the CD82-positive cells are induced in the process of differentiation of stem cells into pancreatic islet cells, can be confirmed by collecting a cell culture sample over time in the differentiation induction process, and then by detecting the presence or absence of the expression of a CD82 protein on the cell surface.

With regard to the period for separation of the CD82-positive cells in the differentiation induction process, the CD82-positive cells may be separated, after induction of the CD82-positive cells has been confirmed by the aforementioned method. Since the period for separation of the CD82-positive cells is different depending on the differentiation induction method or culture conditions, it cannot be univocally specified. The period for separation of the CD82-positive cells is, for example, the 8th to 30th days, preferably the 15th to 27th days, and more preferably 20th to 24th days, after completion of the ordinary differentiation induction treatment of stem cells into pancreatic islets.

When an English translation of the present description includes singular terms with the articles "a," "an," and "the," these terms include not only single items but also multiple items, unless otherwise clearly specified from the context.

Hereinafter, the present invention will be further described in the following examples. However, these examples are only illustrative examples of the embodiments of the present invention, and thus, are not intended to limit the scope of the present invention.

### Examples

### 1. Materials and Methods

### 1-1. Cell Culture

Undifferentiated iPS cells were adhered onto a Matrigel-coated dish in an incubator at 37°C in 5% CO₂, and were then cultured. As maintenance media, mTeSR1 (VERITAS) and StemFlex (Thermo Fisher) were used. The human iPS cell lines, DKI hIveNry #9-15 (Yzumi et al., Scientific Reports 6: 35908, 2016) and TK-D-4M (acquired from Stem Cell Bank, THE INSTITUTE OF MEDICAL SCIENCE, THE UNIVERSITY OF TOKYO), were used in all experiments. EP cells were prepared by using a method obtained by modifying the known protocols (D'Amour et al., Nat Biotechnol. 24: 1392-1401, 2006. Kroon et al., Nat Biotechnol. 26: 443-452, 2008. Rezania et al., Diabetes. 61: 2016-2029, 2012. Rezania et al., Nat Biotechnol. 32: 1121-1133, 2014. Pagliuca et al., 159:428-439 2014. etc.). Specific procedures are described below.

In order to induce differentiation of cells into pancreatic islet cells, human iPS cells (HiPSC) were seeded into StemFlex medium, so that the cells became 80% confluent, and thereafter, the cells were cultured. In the stage of cell differentiation (see Figure 1a), the cells were cultured in media each having a different composition.
- Day 1 of the culture (Stage 1):
   RPMI 1640 medium (FUJIFILM) + 100 ng/ml activin A (PeproTech) + 10 µM CHIR99021 (FUJIFILM)
- Days 2 to 4 of the culture (Stage 2):
   RPMI 1640 medium + 100 ng/ml activin A + 10 % B27 supplement (GIBCO)
- Days 5 to 7 of the culture (Stage 3A):
   DMEM (high-glucose, FUJIFILM) + 50 µM FGF10 (PeproTech) (only Day 5) + 0.25 µM Sant1 (SIGMA) + 700 µM EC23 (Reinnervate) + 6 µM SB431542 + 1 µM Dorsomorphin (FUJIFILM) + 10 % B27 supplement
- Days 8 to 4 of the culture (Stage 3B):
   Medium in Stage 3A + 5 µM RepSox (Abcam)
- Days 15 to 25 of the culture (Stage 4):
   DMEM (high-glucose) + 50 µM FGF10 (only Day 15) + 0.25 µM Sant1 + 1 µM Dorsomorphin +5 µM RepSox + 50 ng/ml IGF-1 (PEPTIDE INSTITUTE, INC.) + Exendin-4 (PEPTIDE INSTITUTE, INC.) + 10 µM DAPT (Tokyo Chemical Industry Co., Ltd. ) + 10 µM Folskolin (FUJIFILM) + 10 % B27 supplement
- Days 26 to 36 of the culture (Stage 5):
   DMEM/F12 (FUJIFILM) + 10% B27 supplement + 0.5 mM HEPES (GIBCO) + 1x PSG (GIBCO) + 2 µM Nicotinamide (FUJIFILM) + 55 µM β-mercaptetol (GIBCO) + 50 ng/ml IGF-1 (FUJIFILM) + Exendin-4 (PEPTIDE INSTITUTE, INC.) + GLP1 (PEPTIDE INSTITUTE, INC.) + 5 µM RepSox + 0.25 µM Sant1 + 2 nM Caspase-3 Inhibitor Z-DEVD-FMK (R & D) + 10 µM Folskolin.

The analysis of the cells was carried out on Day 36 or later. The cells were maintained in the medium of Stage 5 before the use thereof.

The Min6 mouse β cell line (JP Patent Publication (Kokai) No.2002-125661 A) was adhered and cultured, as described above.

### 1-2. Glucose-Stimulated Insulin Secretion (GSIS)

The isolated human pancreatic islets or the differentiation-induced pancreatic islet cells were sampled. Cell clusters were washed twice with a Krebs-ringer solution, and were then pre-incubated in a low-concentration (2.8 mM) glucose Krebs-ringer solution for 2 hours. The cell clusters were washed twice with a Krebs-ringer solution that did not contain glucose, and were then incubated in a low-concentration glucose Krebs-ringer solution for 45 minutes. After that, the supernatant was collected. Subsequently, the supernatant was incubated in a high-concentration glucose (28 mM) Krebs-ringer solution for 45 minutes, and the supernatant was then collected. In some cases, the cells were again incubated in a low-concentration glucose Krebs-ringer solution for 45 minutes. Finally, the cell clusters were incubated in a low-concentration glucose Krebs-ringer solution containing 2 mM glucose and 30 mM KCl for 45 minutes (depolarization challenge), and the supernatant was then collected. The supernatant sample containing the secreted insulin was detected by Human Ultrasensitive C-peptide ELISA and Human C-peptide ELISA (Mercodia).

### 1-3. Preparation of Human-Derived Pancreatic Islet Cells

The use of human pancreatic islets was approved by the Health Research Ethics Committee of the University of Alberta. The use of human pancreatic islets was approved by Health Research Ethic Committee, the University of Tokyo.

The human pancreatic islets were incubated in 1 × TrypLE^{™} Select (1X) at 37°C for 10 minutes, immediately after the arrival thereof. Thereafter, the pancreatic islets were dissociated by pipetting. The cells were separated using the cell sorter MoFlo XDP (Beckman coulter). HUVEC cells were added to the separated pancreatic islet cells at a ratio of 1 : 1, and the mixed cells were then added onto a low-adsorptive 96-well plate (Sumitomo Bakelite Co., Ltd.) at a rate of 20,000 cells/well. As a medium, a phenol red-free RPMI medium (MMM) supplemented with 10% FBS (GIBCO) and 1 % PSG (GIBCO) was used. The next day, cell clusters were collected.

### 1-4. Immunohistochemistry

In order to carry out an immuno-histochemical analysis, cell clusters of pancreatic islets were fixed with 4 % PFA at 4°C for 1 hour, and were then washed. The resultants were embedded in paraffin to produce sections of the pancreatic islet cell clusters. The produced sections were blocked with PBS + 10% donkey serum (Dako) at room temperature for 1 hour, and were then incubated in a blocking buffer containing a primary antibody at 4°C overnight, followed by washing with PBS. The secondary antibody was incubated at 37°C for 2 hours, and was then washed with PBS. Representative images were taken using an Olympus FV3000 confocal microscope.

For whole-mount staining, cell clusters were fixed with 4% PFA at room temperature for 1 hour, and were then washed. Before performing the staining, the cell clusters were treated with 0.5% Triton solution at room temperature for 1 hour. The clusters were stained by the same method as the staining of tissue sections.

### 1-5. Flow Cytometry and Cell Sorting

Differentiated cell clusters or pancreatic islets were dispersed in a single cell suspension by incubation in TrypLE^{™} Express at 37°C for 10 minutes.

For cell sorting, cells were re-suspended in a DMEM medium containing 10% FBS. Subsequently, the cells were re-suspended in a blocking buffer containing a primary antibody, and were then incubated at 4°C for 10 minutes. Thereafter, the cells were washed with a medium twice, and were then incubated with a secondary antibody at 4°C for 10 minutes. Thereafter, the cells were washed twice, and were then sorted using MoFlo XDP. The results were analyzed using the FlowJo software. In FACS requiring intracellular staining, cells were prepared using the BD Cytofix/Cytoperm^{™} Kit (BD Biosciences).

### 1-6. RNA Extraction and Real-Time qPCR

Total RNA was extracted using the RNeasy Micro Kit (Qiagen). cDNA was prepared using the TAKARA PrimeScript^{™} II 1st strand cDNA Synthesis Kit. Real-time PCR measurement was performed using each primer and 0.125 × SYBR Green I (Life Technologies). The data are presented as a mean expression level ±SEM. Relative gene expression was determined using the expression of GAPDH as a house keeping gene.

### 1-7. Gene Expression Analysis Using Microarray

Ngn3-high expressing cells and Ngn3-low expressing cells, which had been purified using a cell sorter, were each subjected to a gene expression analysis using a microarray. Immediately after the purification, total RNA was isolated from the cells using QIAshredder (Qiagen) and RNeasy Mini Kit (Qiagen). Reverse transcription and amplification of the total RNA were performed using the Low Input Quick Amp Labeling Kit (Agilent). Hybridization was performed with SurePrint G3 Human Gene Expression v3 8x60K Microarray kit (Agilent), and was scanned after staining. The microarray data were analyzed by using the functional annotation of DAVID (https://david.ncifcrf.gov/home.jsp). Enriched functions were analyzed, while a P value of 0.05 or less was considered to be significant.

### 1-8. Knockdown of CD82 Using siRNA in Differentiated Human iPS Cells

CD82-positive cells and Ngn3-positive cells were transfected with 20 nM CD82 Stealth RNAi^{™} Oligo (Thermo Fisher) or Block iT Fluorescent Oligo as a control (Thermo Fisher), using Human Stem Cell Nucleofector^{™} Kit 1 (Lonza). On the day following the transfection, the medium was exchanged with another one. After 10 days, glucose-stimulated insulin secretion assay and immunofluorescent staining were performed on the cells.

### 1-9. Inhibition Experiment of CD82 in CD82-Positive and Ngn3-Positive Cells

CD82-positive and Ngn3-positive cells were purified using MoFlo XDP. Thereafter, an anti-CD82 mAb 4F9 antibody and a CD82 antibody LS-C742189 (LSbio) used as neutralizing antibodies or mouse IgG were added at a concentration of 10 mg/ml into a medium, and the cells were then cultured in the medium for 3 days. Thereafter, the cells were cultured in a Stage 5 medium for 7 days. After 10 days, GSIS analysis was carried out.

### 2. Results

### 2-1. Induction of Differentiation of Ngn3-High Expressing Cells into Pancreatic Endocrine Cells

In order to isolate pancreatic endocrine cells, Ngn3-mcherry serving as a pancreatic endocrine progenitor cell (EPC) marker and INS-Venus serving as a β cell marker were double knocked in iPS cells, and the resulting iPS cells were then allowed to differentiate into EP cells (EPCs) by using the multistage protocols of the inventors (Figure 1a). On Day 22 after the differentiation, the EP stage cells were analyzed according to flow cytometry based on mcherry expression intensity. It was found that the EP cells comprised approximately 12.3% of the Ngn3-high expressing cell population and approximately 10.9% of the Ngn3-low expressing cell population (Figure 1d). In order to confirm the mcherry signal specificity of the EP cells, gene expression analysis was performed on the sorted cell fractions. From the results of qRT-PCR analysis, it became clear that Ngn3-high expressing cell fraction-derived cells express the EP stage cell marker genes, PDX1, Ngn3, NeuroD1, Nkx6.1 and MafA, at higher expression levels, than Ngn3-negative cell fraction-derived and Ngn3-low expressing cell fraction-derived cells (Figure 1e). These results suggest that a large number of Ngn3-high expressing cells be comprised in the EP stage cell population. In addition, these results are consistent with the previous results obtained using purified Ngn3-positive cells (H Liu et al., Cell Res. Oct; 24(10): 1181-200. 2014).

Next, in order to evaluate the ability of the isolated Ngn3-high expressing EP cells and Ngn3-low expressing EP cells to differentiate into mature pancreatic endocrine cells, these cells were allowed to differentiate into pancreatic endocrine cells according to the aforementioned multistage protocols (Figure 1b). The Ngn3-high expressing cells, Ngn3-low expressing cells, Ngn3-negative cells, and non-sorted cells were cultured in a suspension culture system for 10 days. As a result, it was observed that cell clusters having a uniform shape were formed in the culture systems of the Ngn3-high expressing cells and the Ngn3-low expressing cells (Figure If). The insulin secretion of these cell clusters was analyzed using the secreted amount of a C-peptide as an indicator. As a result, it became clear that the insulin secretion of the Ngn3-high expressing cell-derived cell cluster is higher than the insulin secretion of the Ngn3-low expressing cell-derived cell cluster (Figure 1g). In order to determine the percentage of mature β cells in the cell cluster, the percentage of Nkx6.1 and insulin co-expressing cells (Nkx6.1/INS) was analyzed by flow cytometry (Figure 1h). As a result, the percentage of the Nkx6.1/INS double positive cells in the Ngn3-high expressing cell cluster was 25.1%, whereas the percentage of the Nkx6.1/INS double positive cells in the Ngn3-low expressing cell cluster was 12.2%.

The aforementioned results suggest that the EP stage cells can be purified by isolation (separation) of the Ngn3-high expressing cells, and that the purified cells be able to differentiate into insulin secreting β cells.

### 2-2. Studies regarding Function of CD82 as Pancreatic Endocrine Progenitor Cell Marker

In order to identify a marker gene that is strongly expressed in Ngn3-high expressing cells, the gene expression patterns of Ngn3-high expressing cells and Ngn3-low expressing cells were analyzed using a microarray. As a result of the microarray analysis, it became clear that pancreatic development marker genes tend to be expressed at higher levels in the Ngn3-high expressing cells than in the Ngn3-low expressing cells (Figure 2a). The EP stage markers CD200 and CD142 (F3) were expressed in the Ngn3-high expressing cells, but the EP markers Susd2 and GP2 were expressed at low levels in the Ngn3-high expressing cells.

Among genes that are expressed in the Ngn3-high expressing cells, 517 genes that are expressed in the Ngn3-high expressing cells at a higher level (5 fold or more) than in the Ngn3-low expressing cells were analyzed. GO-term analysis showed that signal peptide-related, membrane protein-related, and glucose homeostasis-related genes are expressed at high levels in the Ngn3-high expressing cells (Figure 2b). Moreover, the Ngn3-high expressing cells were rich in insulin secretion-related factors.

Next, in order to select a cell membrane marker strongly expressed in the Ngn3-high expressing cells, the genes were analyzed by applying a filter with the GO terms 'membrane' and 'signal.' As a result, 72 genes were hit. Based on a list of these 72 genes, the expression status on the cell surface was analyzed according to FACS using commercially available antibodies. As a result, CD82 was found. CD82 belongs to the tetraspanin protein family as a 4-transmembrane protein and is known as a suppressor of pancreatic cancer and the like (WM Liu et al., Cancer Lett. Aug 28; 240(2): 183-94. 2005: CM Termini et al., Front Cell Dev Biol. Apr 6; 5: 34. 2017, etc.).

In order to examine the abundance percentage of CD82-positive cells in the process of differentiation into the pancreas, FACS analysis was carried out. CD82 was hardly expressed in the cells on the 6th day (Day 6) and the 9th day (Day 9) after initiation of induction of the differentiation, but was strongly expressed in the cells on the 22nd day (Day22) (Figure 2c). In addition, these results were not dependent on a difference in cell lines. In order to verify the expression of CD82 in EP cells, the qRT-PCR analysis of CD82+/- fractions was carried out (Figure 2d). When compared with in the CD82-negative fraction cells, it was observed that pancreatic endocrine progenitor cell markers, such as PDX1, Nkx6.1, Mnx1, Pax4, MafA, Ins and NeuroD1, and particularly, pancreatic β cell markers, were expressed at high levels in the CD82-positive fraction cells. Moreover, in the CD82-positive cell fraction and the CD82-negative cell fraction, the expression of PDX1 and Nkx6.1 was examined by FACS (Figure 2e). As a result, the abundance percentage of Nkx6.1/PDX1 double positive cells was 0.84% in CD82-negative cells, whereas it was 21.1% in CD82-positive cells (Figure 2e). Furthermore, in the cells on Day 22, all of the CD82-positive cells were PDX1- and NeuroD1-positive cells. However, not all PDX1-expressing cells and NeuroD1-expressing cells expressed CD82 (Figure 2f). These results suggest that the CD82-positive cells be likely to be a specific subset of pancreatic endocrine progenitor cells.

From the aforementioned results, it was suggested that a subset of endocrine progenitor cells be preferentially concentrated in the CD82-positive cells.

2-3. Studies regarding Induction of Differentiation of CD82-Positive Cells into Mature Endocrine Cells

Next, in order to study whether CD82-positive cells can differentiate into mature pancreatic endocrine cells, CD82-positive cells and CD82-negative cells, which had been purified with a cell sorter, were subjected to a maturation culture. As with the Ngn3-positive cells, the CD82-positive cells formed uniform cell clusters (Figure 3a). In order to examine the percentage of endocrine progenitor cell comprised in a CD82-positive cell fraction and a CD82-negative cell fraction, flow cytometric analysis was carried out (Figure 3b). It was found that 33% of C-Pep (C-PEPTIDE)/PDX1 double positive cells and 38.1% of C-pep/ND1 (NEUROD1) double positive cells were comprised in the CD82-positive cell fraction. In contrast, 1.65% of C-Pep/PDX1 double positive cells and 1.6% of C-Pep/ND1 double positive cells were comprised in the CD82-negative cell fraction. In addition, 5.71% of GCG (glucagon)-positive cells were comprised in the CD82-positive cell fraction, whereas 1.46% of the GCG-positive cells were comprised in the CD82-negative cells.

It has been reported that many of the previously reported pancreas differentiation protocols generate immature polyhormone cells simultaneously expressing glucagon and insulin (E Kroon et al., Nat Biotechnol. Apr; 26(4): 443-52. 2008: JE Bruin et al., Stem Cell Res. Jan; 12(1): 194-208. 2014: MC Nostro et al., Development 138, 861-871, 2011.). The abundance percentage of INS/GCG double positive cells was 2.32% in a CD82-positive cell-derived cell cluster and was 0.97% in a CD82-negative cell-derived cell cluster.

The aforementioned results demonstrate that a cell cluster comprising mature CD82-positive cells comprises almost no polyhormone cells and is composed of mature β cells and other mature endocrine cells. In contrast, it was found that a CD82-negative cell-derived cell cluster comprises PDX1-positive cells but hardly comprises β cells.

Moreover, the localization of endocrine cells in the cell cluster was examined by immunostaining (Figure 3c). As a result, it was found that the CD82-positive cell-derived cell cluster consisted of monohormonal β cells, α cells and δ cells (somatostatin (SST)-producing cells). In contrast, cavities were formed in the CD82-negative cell-derived cell cluster, and there was almost no INS-positive cells in the cell cluster.

### 2-4. Comparison between CD82 and Known Pancreatic Endocrine Cell Markers

To date, a plurality of gene markers, such as CD200, GP2 and susd2, have been reported as specific markers to pancreatic islet progenitor cells (H Liu et al., Cell Res. Oct; 24(10): 1181-200. 2014: J Ameri et al., Cell Rep. Apr 4; 19(1): 36-49. 2017: OG Kelly et al., Nat Biotechnol. Jul 31; 29(8): 750-6. 2011). It has been demonstrated that cells that express these markers are able to differentiate into mature pancreatic endocrine cells *in vitro* or *in vivo.*

Hence, a comparison was made in terms of the expression levels of CD82 and other marker genes in EP stage cells (Figure 4a). From the results of FACS analysis, it was found that, in the EP stage cells, CD82 is not expressed in CD200-, CD142-, GP2- and Susd2-positive cells or is expressed only in some of the cells. These results suggest that the CD82-positive cells be cells different from the previously reported pancreatic endocrine progenitor cell marker-positive cells.

The expression levels of the previously reported EP marker genes in the sorted cell populations (i.e. cell populations sorted with CD142, CD200 and CD82) were analyzed by a qRT-PCR method. As a result, the expression levels of the EP marker genes in the CD82-positive cells were equivalent to the expression levels thereof in the cell populations sorted with CD142 and CD200 (Figure 4b).

Subsequently, CD142, CD200, Susd2-expressing cells, and CD82-expressing cells were subjected to a maturation culture according to the protocols shown in Figure 1a, and the secretion amount of a C-peptide was then measured. As a result, being consistent with the previous reports, glucose level-responsive insulin secretion was found in the cells sorted with CD 142, CD200 and Susd2. However, between each marker-negative cells and each marker-positive cells, there was not found a large difference in terms of the secretion amount of a C-peptide. In contrast, it was found that the CD82-positive cells have glucose level-responsive insulin secretion that is significantly higher than that of CD82-negative cells. These results demonstrate that, differing from cell fractions recovered with the previously reported pancreatic progenitor cell markers (CD142, CD200 and Susd2), the CD82-positive cell fraction is more efficiently induced to differentiate into β cells as a result of having been subjected to a maturation culture (Figure 4c).

### 2-5. CD82-Positive Cells in Mature Pancreatic Islets

It has been known that CD82 is highly expressed in pancreatic cancer (Guo X, Cancer Res. 56: 4876-4880, 1996), and it has been demonstrated that CD82 is involved in various cell functions, such as suppression of the migratory ability of cells. In addition, CD82 is also expressed in normal pancreas, but the role thereof has not yet been determined. In order to examine the expression pattern of CD82 in normal pancreatic islets, CD82 expression in human adult pancreatic islet sections was analyzed. CD82 stained the islet as a whole and did not distinguish various types of endocrine cells from one another (Figure 5a). Moreover, from the images stained with the CD82 antibody, it was shown that CD82 is mainly present in the cytoplasm and the cell membrane. These results were consistent with the report regarding other tissues (R. Rotterud et al., Histol Histopathol 22: 349-363, 2007).

The role of CD82-positive cells in human pancreatic islet cells was analyzed (Figure 5b). The CD82-positive cells accounted for 30% to 40% in the entire pancreatic islet cells. Cells expressing a C-peptide serving as a mature β cell marker were present at a percentage of 40.2% in CD82-negative cells, and at a percentage of 65.02% in CD82-positive cells. Among these cells, cells that co-express UCN3 and C-peptide were observed at a percentage of 64.6% in CD82-positive cells, and at a percentage of 15.1% in CD82-negative cells (Figure 5c). These results suggest that pancreatic β cells having more mature properties be rich in CD82-positive cell fractions.

The glucose-stimulated insulin secretion (GSIS) of the CD82-positive cell fraction and the CD82-negative cell fraction was measured. Since the isolated pancreatic islet cells do not have insulin secretion, a niche necessary for the cell function needs to be reproduced. To date, it has been reported that HUVEC cells and mesenchymal stem cells (MSC) have the function of aggregating pancreatic islet tissues and pancreatic islet cells (Takahashi et al., Cell Reports 23, 1620-1629, 2018). Based on these findings, the isolated CD82 positive/negative cells and HUVEC cells were co-cultured at a ratio of 1 : 1. Twenty-four hours after the culture, the cells formed cell clusters (Figure 5d).

The pseudo pancreatic islet clusters derived from the CD82-positive cells secreted insulin in a high glucose concentration in an amount approximately two times as large as in a low glucose concentration (Figure 5e). In contrast, CD82-negative cell-derived pancreatic -islet clusters showed a small amount of insulin secretion, but did not show the effect of enhancing insulin secretion due to a high glucose concentration. These results show that the CD82-positive cells are able to differentiate into functional β cells, more efficiently than the CD82-negative cells. When the CD82 positive/negative cells were subjected to immunostaining, it became clear that the CD82-positive cell clusters are composed of β cells, α cells and δ cells, whereas the CD82-negative cell clusters comprise almost no β cells.

### 2-6. Studies regarding Expression Period of CD82

In order to confirm the expression period of CD82 in the development stage, the expression pattern of CD82 was examined in the mouse pancreatic development process. CD82-positive cells were specifically detected in the PDX1-positive pancreatic portion of E18.5 mice (Figure 6, the upper panels). Notably, such CD82-positive cells could not be detected in any site of the pancreas before E16.5. In mouse fetus, Ngn3-positive cells appear after E13.5, but the expression of CD82 is not consistent with this period. In addition, in adult mouse pancreas, CD82 expression was detected in almost all pancreatic islet cells (Figure 6, the lower panels). These findings suggest that CD82 be expressed *in vivo* in pancreatic islet cells that are nearly matured.

### 2-7. Influence of CD82 on Insulin Secretion of β Cells

Next, whether or not CD82 has influence on the function of iPS cell-derived pancreatic islets, in particular, the function of β cells was examined. The expression of CD82 in Ngn3-high expressing cells was knocked down, and maturation of the cells to β cells was then induced. Thereafter, GSIS was evaluated. As a result, it was found that when CD82 is knocked down, glucose responsiveness observed in a control is lost (Figure 7a). Moreover, an anti-CD82 mAb 4F9 antibody known to inhibit the VEGF-dependent migration function and proliferating ability of mesothelial cells in HUVEC cells (Iwata et al., Eur. J. Immunol. 32: 1328-1337, 2002 Nomura et al., Biochemical and Biophysical Research Communications 474, 111e117, 2016) was used to attempt to inhibit the function of CD82. As a result, it was found that glucose responsiveness observed in a control is lost, as with the results of siRNA (Figure 7b, antibody 1). Furthermore, the same results were obtained even in the case of using a commercially available CD82 inhibitory antibody (CD82 antibody LS-C742189) (Figure 7b, antibody 2). Interestingly, even if an inhibitory antibody was added to the cells after completion of the maturation culture, there was no influence on the glucose level-responsive insulin secretion.

From the aforementioned results, it is considered that the expression of CD82 is likely to regulate the GSIS of β cells in the differentiation induction process.

### Industrial Applicability

Since the pancreatic progenitor cells (or cell group) of the present invention can be prepared by being separated from undifferentiated cells or unnecessary cells, the efficiency of the maturation culture of the pancreatic progenitor cells is improved, and also, the possibility of canceration can be avoided. Therefore, it is expected that the pancreatic progenitor cells (or cell group) of the present invention will be utilized in the medicinal field such as transplantation therapy.

## Claims

1. Pancreatic progenitor cells appearing in the process of differentiation of stem cells into pancreatic islet cells, which are **characterized in that** the pancreatic progenitor cells are positive to CD82.

2. Pancreatic progenitor cells according to claim 1, which are **characterized in that** the stem cells are pluripotent stem cells.

3. A cell group comprising the pancreatic progenitor cells according to claim 1 or 2.

4. An agent for treating blood glucose level impairment, wherein the agent comprises the pancreatic progenitor cells according to claim 1 or 2.

5. The treatment agent according to claim 4, which is **characterized in that** the blood glucose level impairment is hyperglycemia.

6. A method for preparing pancreatic progenitor cells and/or mature pancreatic β cells, wherein the method comprises separating CD82-positive cells that appear in the process of differentiation of stem cells into pancreatic islet cells.

7. The method according to claim 6, which is **characterized in that** the stem cells are pluripotent stem cells.
